# EUROPÄISCHE PATENTSCHRIFT

(11) **EP 2 845 393 B1**
(45) Veröffentlichungstag und Bekanntmachung des Hinweises auf die Patenterteilung: **31.01.2018**
(21) Anmeldenummer: 13721631.3
(22) Anmeldetag: 29.04.2013
(51) Int. Cl.: H04R 1/10, H04R 31/00, A61F 11/06

(54) **GEHÖRSCHUTZ-OTOPLASTIK FÜR AKTIVEN GEHÖRSCHUTZ**
EARPLUG-MOULD FOR ACTIVE HEARING PROTECTION
MOULAGE D'UNE OREILLETTE AVEC PROTECTION AUDITIVE ACTIVE

(30) Priorität: 03.05.2012 DE 102012207375
(43) Veröffentlichungstag der Anmeldung: 11.03.2015
(73) Patentinhaber: Bachmaier GmbH & Co. Kg, 83486 Ramsau (DE)
(72) Erfinder: KUBICKE, Bernhard, 83486 Ramsau (DE); KUBICKE, Fabian, 83486 Ramsau (DE)
(74) Vertreter: Winter, Brandl, Fürniss, Hübner, Röss, Kaiser, Polte - Partnerschaft mbB
(86) Internationale Anmeldenummer: PCT/EP2013/058887
(87) Internationale Veröffentlichungsnummer: WO 2013/164305

(56) Entgegenhaltungen:
- EP-A1- 1 629 805
- EP-A2- 1 819 190
- DE-U1- 20 018 829
- US-A1- 2006 045 297

## Beschreibung

Die Erfindung betrifft eine Gehörschutz-Otoplastik für den aktiven Gehörschutz, wie sie beispielsweise in Ulrich Vogt:" Otoplastik - Die individuelle Otoplastik zur Hörgeräteversorgung und als persönlicher Gehörschutz im Lärm", Band 2 der wissenschaftlichen Fachbuchreihe, Akademie für Hörgeräte-Akustik, Lübeck 1998, Median-Verlag von Killisch-Horn GmbH beschrieben ist.

Dabei geht man herkömmlicher Weise so vor, dass durch Abformung des Ohres unter Einschluss der ersten Gehörgangs-Krümmung das Gehäuse der Gehörschutz-Otoplastik ausgebildet und anschließend in den Gehäusekörper die erforderlichen Komponenten des Aktiv-Gehörschutzes, wie z.B. Lautstärkesteller, Batterie, Mikrofon, Elektronik-Chip und Schall-Wandler an denjenigen Stellen eingebettet werden, an denen sich - je nach vorliegenden anatomischen Verhältnissen - ausreichend Platz findet.

Aus dem Dokument EP 1 819 190 A2 ist bereits ein System bekannt, bei dem in einen Otoplastikkörper selektiv ein Passiv-Schallschutzfilter und eine Aktiv-Gehörschutz-Komponente eingesetzt werden kann. Allerdings bildet die Ausnehmung im Otoplastikkörper nicht die Schnittstelle für die Aktiv-Gehörschutz-Komponente. Vielmehr wird die Aufnahme für das Passiv-Schallschutzfilter in einem separaten, d.h. zusätzlichen Gehäuseteil ausgebildet.

Ferner ist aus dem Dokument US 2006/0045297 A1 eine Otoplastik mit zugehörigem Herstellungsverfahren bekannt geworden, wobei für die Bestückung und Aufrüstung der Otoplastik mit verschiedenen Funktionskomponenten eine einheitliche Gehäuseschale verwendet wird. Soweit ein Passiv-Schallschutzfilter verwendet wird, ist dieser auf der dem Gehörgang zugewandten Seite in den Otoplastikkörper eingesetzt.

Solche herkömmlichen Gehörschutz-Otoplastiken haben den Nachteil einer verhältnismäßig aufwändigen Fertigung, so dass der Erfindung die Aufgabe zugrunde liegt, die Gehörschutz-Otoplastik für den aktiven Gehörschutz derart weiterzubilden, dass sich unter Beibehaltung eines guten Tragekomforts eine wesentlich wirtschaftlichere Herstellung ergibt.

Diese Aufgabe wird durch die Merkmale des Anspruchs 1 gelöst. Erfindungsgemäß wird ein neuartiger Gehäusemodul für eine Aktiv-Gehörschutz-Komponente geschaffen, der einen Gehäuseabschnitt hat, welcher hinsichtlich seiner Außenabmessungen identisch zu einem Gehäuseabschnitt eines Passiv-Schallschutzfilters ausgebildet ist, für das im Otoplastikkörper auf dessen dem Gehörgang abgewandten Stirnseite eine Ausnehmung zur verliersicheren, passgenauen und vorzugsweise bündig versenkten Aufnahme des Passiv-Schallschutzfilters vorgesehen ist. Damit wird erfindungsgemäß die für das Passiv-Schallschutzfilter vorhandene Ausnehmung im Otoplastikkörper als Schnittstelle für die Aktiv-Gehörschutz-Komponente genutzt, die somit mit einem einheitlichen, von der Anatomie des die Otoplastik tragenden Nutzers unabhängig gestalteten Gehäuse und damit wesentlich wirtschaftlicher herstellbar ist. Weil die die Universal-Schnittstelle bildende Ausnehmung im Otoplastikkörper eine kreiszylindrische Vertiefung hat, an die sich bodenseitig eine weitere im Durchmesser reduzierte, hinterschnittene Ausnehmung mit Kreisquerschnitt anschließt, ergibt sich eine besonders einfache Schnittstelle zur Ankopplung der Aktiv-Gehörschutz-Komponente. Eine wesentliche weitere Verbesserung der Wirtschaftlichkeit ergibt sich auch dadurch, dass ein einziger durch Abformung des Ohres herzustellender Otoplastik-Körper genügt, um einen Passiv- und einen Aktiv-Gehörschutz bereit zu stellen. Auch die Montage bzw. die Umrüstung der Otoplastik vom Passiv- zum Aktiv-Gehörschutz und umgekehrt wird erfindungsgemäß sehr einfach, indem die Elastizität des Otoplastikkörpers in vorteilhafter Weise ausgenützt wird.

Vorteilhafte Weiterbildungen sind Gegenstand der Unteransprüche.

Es hat sich herausgestellt, dass es durch Verwendung von zwar miniaturisierten, aber gängigen und im Handel ohne Weiteres erhältlichen Komponenten des Aktiv-Gehörschutzes gelingt, das Gehäuse der Aktiv-Gehörschutz-Komponente im Wesentlichen zylindrisch auszubilden, wobei der Durchmesser hinter den stirnseitigen Abmessungen des Otoplastikkörpers zurückbleiben kann. Auf diese Weise wird auch das Gewicht der Aktiv-Gehörschutz-Komponente ausreichend klein, so dass die relativ klein bauende Schnittstelle im Otoplastikkörper ausreicht, um die Aktiv-Gehörschutz-Komponente selbst bei sportlicher Betätigung verliersicher am Otoplastikkörper zu halten.

Versuche haben ergeben, dass es dabei ausreicht, die axiale Länge des Gehäuses der Aktiv-Gehörschutz-Komponente so zu gestalten, dass sie etwa der doppelten Tiefe der Ausnehmung im Otoplastikkörper entspricht.

Insbesondere dann, wenn der Otoplastikkörper aus einem weichen und elastischen Material, wie z.B. einem elastischen Silikonkautschukmaterial hergestellt wird, ergeben sich mit der Weiterbildung des Anspruchs 5 besondere Vorteile hinsichtlich des Tragekomforts und der Montage und Demontage der Gehörschutz-Komponenten. Der die kreiszylindrische Vertiefung umgebende ringförmige Gehäuseabschnitt mit vorzugsweise gleichbleibender Wandstärke lässt sich leicht mit der Hand verformen, so dass das Einsetzen und Austauschen der Gehörschutz-Komponenten besonders einfach wird. Die Elastizität des ringförmigen Gehäuseabschnitts kann allerdings gleichzeitig für deren feste Verankerung im Gehäusekörper genutzt werden.

In der Aktiv-Gehörschutz-Komponente sind in der Regel folgende Bauelemente aufgenommen und miteinender verdrahtet: ein Lautstärkesteller, der auch mit einem Programm-Taster kombiniert sein kann, eine Batterie, ein Mikrofon, ein Elektronik-Chip und ein Miniatur-Schall-Wandler.

Während der Miniatur-Schall-Wandler aus physikalischen Gründen radial innerhalb eines hinterschnittenen Konuszapfens der Aktiv-Gehörschutz-Komponente zu liegen kommt, ergibt sich durch die Weiterbildung des Anspruchs 9 eine besonders einfache, weil universell anwendbare Gestaltung des Gehäuses für die Aktiv-Gehörschutz-Komponente.

Weitere vorteilhafte Ausgestaltungen sind Gegenstand der übrigen Unteransprüche.

Nachstehend wird anhand schematischer Zeichnungen ein Ausführungsbeispiel der Erfindung näher erläutert. Es zeigen:
Figur 1 eine schematische in vergrößerter Darstellung gezeigte Seitenansicht der erfindungsgemäßen Aktiv-Gehörschutz-Otoplastik;
Figur 2 eine perspektivische Ansicht der Aktiv-Gehörschutz-Otoplastik nach Figur 1;
Figur 3 eine vergrößerte, maßstabsgetreue Seitenansicht der Aktiv-Gehörschutz-Komponente;
Figur 4 eine Stirnansicht der Aktiv-Gehörschutz-Komponente gemäß Figur 3;
Figur 5 eine schematische Darstellung der Aktiv-Gehörschutz-Komponente zur Erläuterung der Anordnung der im Gehäuse angeordneten Bauelemente; und
Figur 6 eine schematisierte Schnittansicht eines Gehörgangs mit eingesetzter Aktiv-Gehörschutz-Otoplastik.

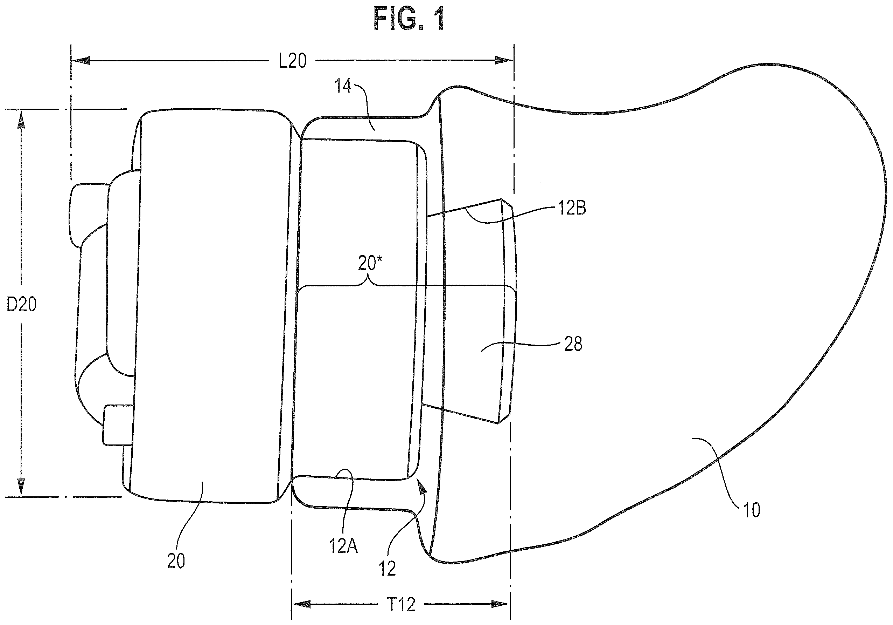

In Figur 1 ist mit dem Bezugszeichen 10 ein Otoplastikkörper bezeichnet, der durch individuelle Ohrabformung unter Einschluss zumindest der ersten Gehörgangs-Krümmung ausgebildet ist. Der Otoplastikkörper 10 ist vorzugsweise aus elastischem Kunststoff, wie z.B. einem Silikonkautschuk-Material gebildet.

Auf der dem Gehörgang abgewandten Seite ist im Otoplastikkörper 10 stirnseitig eine Ausnehmung 12 ausgebildet, in der verliersicher und passgenau und vorzugsweise bündig versenkt ein nicht näher dargestelltes Passiv-Schallschutzfilter aufgenommen werden kann. Die Ausnehmung 12 wird als Schnittstelle für eine mit dem Bezugszeichen 20 versehene Aktiv-Gehörschutz-Komponente genutzt, die mit einem hinsichtlich ihrer Außenabmessungen identisch zum Passiv-Schallschutzfilter ausgebildeten Gehäuseabschnitt 22 in die Ausnehmung 12 einsetzbar ist.

Das Gehäuse der Aktiv-Gehörschutz-Komponente 20 ist im Wesentlichen zylindrisch ausgebildet, wobei der größte Durchmesser D20 hinter den stirnseitigen Abmessungen des Otoplastikkörpers 10 zurückbleibt.

Die Ausnehmung 12 im Otoplastikkörper 10 ist von einer kreiszylindrischen Vertiefung 12A gebildet, an die sich bodenseitig eine weitere im Durchmesser reduzierte, hinterschnittene Ausnehmung 12B mit Kreisquerschnitt anschließt. Das Gehäuse der Aktiv-Gehörschutz-Komponente 20 ist an dieser Stelle komplementär ausgebildet, allerdings mit einem geringfügigen Übermaß, so dass die Aktiv-Gehörschutz-Komponente formschlüssig und mit elastischem Kraftschluss in der Ausnehmung 12 gehalten werden kann.

Die axiale Länge L20 des Gehäuses der Aktiv-Gehörschutz-Komponente 20 etwa der doppelten Tiefe T12 der Ausnehmung 12 im Otoplastikkörper 10. Wie der Figur 1 entnommen werden kann, ist die kreiszylindrische Vertiefung 12A im Otoplastikkörper 10 von einem ringförmigen Gehäuseabschnitt 14 mit vorzugsweise gleichbleibender Wandstärke umgeben. Die durch das Material des Otoplastikkörpers vorgegebene Elastizität erlaubt es dementsprechend, die Aktiv-Gehörschutz-Komponente 20 durch leichten Druck von Hand auf den ringförmigen Gehäuseabschnitt 14 aus dem Otoplastikkörper 10 zu entfernen und durch einen Passiv-Gehörschutz zu ersetzen, der dann vorzugsweise bündig im Otoplastikkörper 10 sitzt.

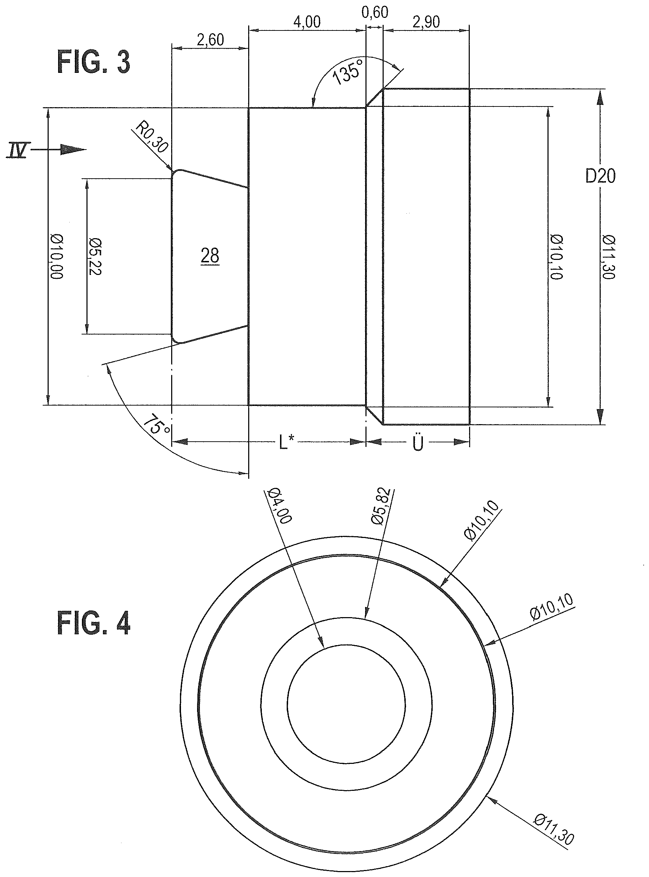

Den Figuren 3 und 4 sind die exakten Maße der Gehörschutz-Otoplastik zu entnehmen. Die axiale Länge beträgt etwa 10 mm, der größte Durchmesser nur 11 mm. Die axiale Länge L* des die Schnittstelle für die Ausnehmung 12 im Otoplastikkörper 10 bildenden Gehäuseabschnitts beträgt nur 6,6 mm.

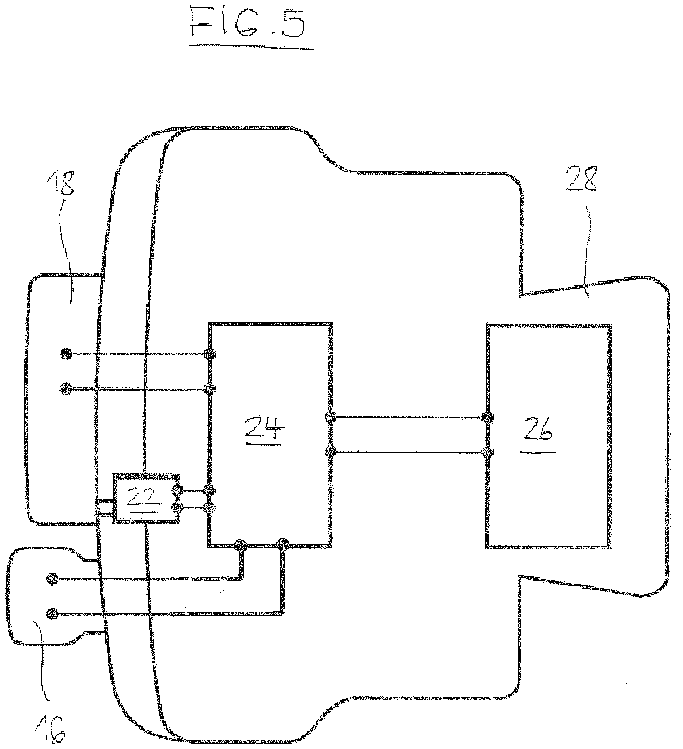

Wie der Figur 5 entnommen werden kann, sind in der Aktiv-Gehörschutz-Komponente 20 folgende Bauelemente aufgenommen und miteinender verdrahtet sind: ein Lautstärkesteller 16, eine Batterie 18, ein Mikrofon 22, ein Elektronik-Chip 24 und ein Miniatur-Schall-Wandler 26.

Der Lautstärkesteller 16 kann mit einem Programm-Taster kombiniert sein, mittels welchem verschiedene auf dem Elektronik-Chip 24 hinterlegte Funktionen oder Programme ausgewählt bzw. angesteuert werden können, um z.B. die Empfindlichkeit, Dämpfung oder andere Funktionalitäten einzelner Bauelemente einzustellen.

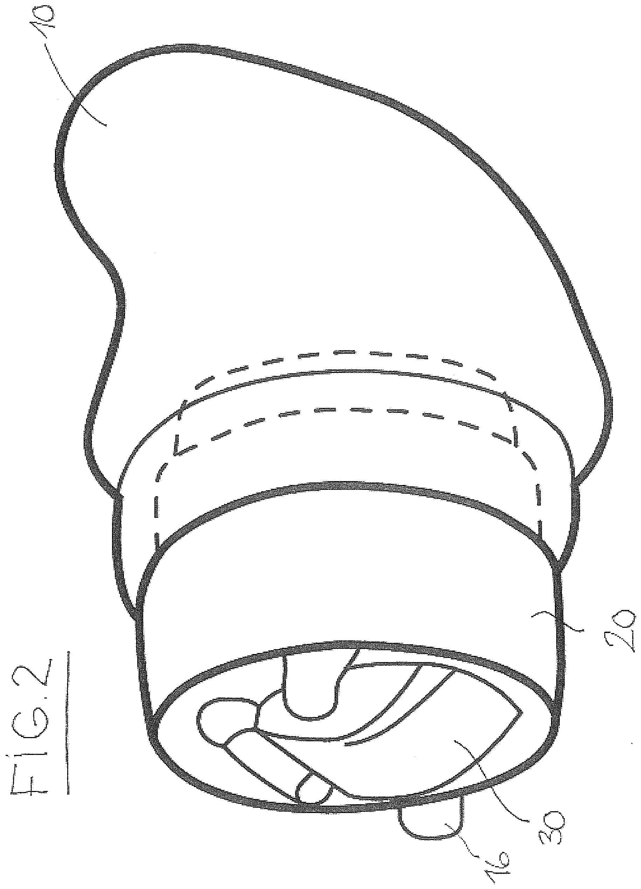

Man erkennt, dass der Miniatur-Schall-Wandler 26 radial innerhalb eines hinterschnittenen Konuszapfens 28 der Aktiv-Gehörschutz-Komponente 20 liegt und dass-wie in Figur 2 erkennbar - ein Batteriefachdeckel 30 und ein Teil des Lautstärkestellers 30 stirnseitig vom Gehäuse der Aktiv-Gehörschutz-Komponente 20 vorstehen.

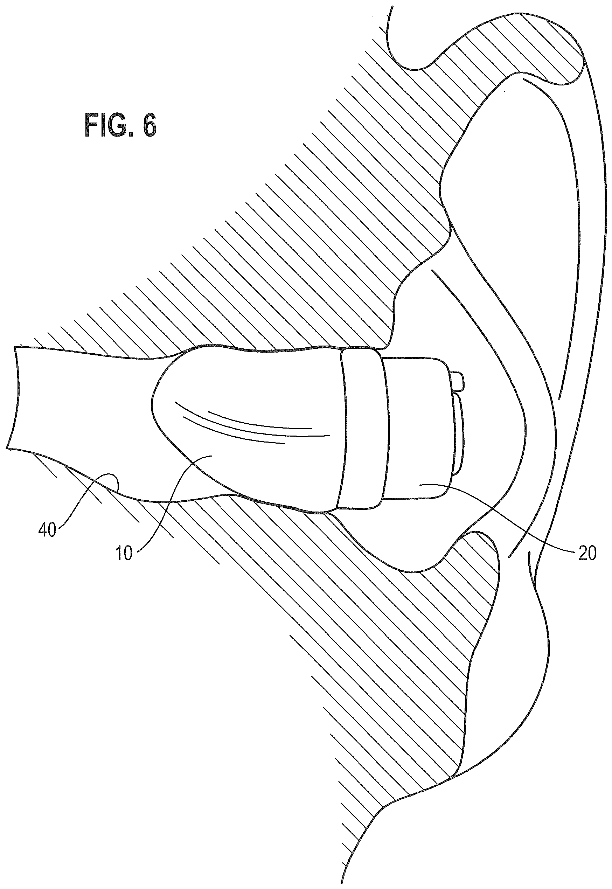

Der Darstellung der Figur 6 kann entnommen werden, dass die Aktiv-Gehörschutz-Otoplastik lediglich um ein Maß Ü - siehe auch Figur 3 - von 3 bis 4 mm vom Gehörgang vorsteht, so dass ein hervorragender Tragekomfort geschaffen wird. Die Aktiv-Gehörschutzkomponente 20 kann als mit der Schnittstelle ausgestatteter Universal-Modul, und damit wirtschaftlicher als bislang gefertigt werden, lediglich der Otoplastikkörper wird individuell durch Ohrabformung hergestellt.

## Patentansprüche

1. Gehörschutz-Otoplastik für den aktiven Gehörschutz, mit einem nach einer Ohrabformung gearbeiteten Otoplastikkörper (10), der auf seiner dem Gehörgang (40) abgewandten Stirnseite eine Ausnehmung (12) zur verliersicheren, passgenauen und versenkten Aufnahme eines Passiv-Schallschutzfilters aufweist, und mit einer Aktiv-Gehörschutz-Komponente (20), **dadurch gekennzeichnet, dass** die Ausnehmung (12), die eine kreiszylindrische Vertiefung (12A) hat, an die sich bodenseitig eine weitere im Durchmesser reduzierte, hinterschnittene Ausnehmung (12B) mit Kreisquerschnitt anschließt, als Schnittstelle für die Aktiv-Gehörschutz-Komponente (20) genutzt wird, wobei die Aktiv-Gehörschutz-Komponente (20) mit einem hinsichtlich ihrer Außenabmessungen identisch zu dem in die Gehörschutz-Otoplastik einsetzbaren Passiv-Schallschutzfilter ausgebildeten Gehäuseabschnitt (20*), der komplementär, allerdings mit einem geringfügigen Übermaß zur Ausnehmung (12) im Otoplastikkörper ausgebildet ist, in die Ausnehmung (12) einsetzbar ist, so dass die Aktiv-Gehörschutz-Komponente (20) formschlüssig und mit elastischem Kraftschluss in der Ausnehmung (12) gehalten werden kann.

2. Gehörschutz-Otoplastik nach Anspruch 1, **dadurch gekennzeichnet, dass** das Gehäuse der Aktiv-Gehörschutz-Komponente (20) im Wesentlichen zylindrisch ist, wobei der Durchmesser (D20) hinter den stirnseitigen Abmessungen des Otoplastikkörpers (10) zurückbleibt.

3. Gehörschutz-Otoplastik nach Anspruch 2, **dadurch gekennzeichnet, dass** die axiale Länge (L20) des Gehäuses der Aktiv-Gehörschutz-Komponente (20) etwa der doppelten Tiefe (T12) der Ausnehmung (12) im Otoplastikkörper (10) entspricht.

4. Gehörschutz-Otoplastik nach einem der Ansprüche 1 bis 3, **dadurch gekennzeichnet, dass** der Otoplastikkörper (10) die kreiszylindrische Vertiefung (12A) mit einem ringförmigen Gehäuseabschnitt (14) mit vorzugsweise gleichbleibender Wandstärke umgibt.

5. Gehörschutz-Otoplastik nach einem der Ansprüche 1 bis 4, **dadurch gekennzeichnet, dass** der Otoplastikkörper (10) aus einem elastischen Silikonkautschukmaterial besteht.

6. Gehörschutz-Otoplastik nach einem der Ansprüche 1 bis 5, **dadurch gekennzeichnet, dass** in der Aktiv-Gehörschutz-Komponente (20) folgende Bauelemente aufgenommen und miteinander verdrahtet sind: ein Lautstärkesteller (16), eine Batterie (18), ein Mikrofon (22), ein Elektronik-Chip (24) und ein Miniatur-Schall-Wandler (26).

7. Gehörschutz-Otoplastik nach Anspruch 6, **dadurch gekennzeichnet, dass** der Miniatur-Schall-Wandler (26) radial innerhalb eines hinterschnittenen Konuszapfens (28) der Aktiv-Gehörschutz-Komponente (20) liegt.

8. Gehörschutz-Otoplastik nach Anspruch 6 oder 7, **dadurch gekennzeichnet, dass** ein Batteriefachdeckel (30) und ein Teil des Lautstärkestellers (16) stirnseitig vom Gehäuse der Aktiv-Gehörschutz-Komponente (20) vorstehen.

9. Gehörschutz-Otoplastik nach einem der Ansprüche 1 bis 8, **dadurch gekennzeichnet, dass** die Aktiv-Gehörschutz-Komponente (20) eine axiale Länge von etwa 10 mm und einen Durchmesser von maximal 11 mm hat.

10. Gehörschutz-Otoplastik nach einem der Ansprüche 1 bis 9, **dadurch gekennzeichnet, dass** das Passiv-Schallschutzfilter in der Ausnehmung (12) bündig versenkt aufnehmbar ist.

## Claims

1. Hearing-protection earmold for active hearing protection, having an earmold body (10) formed in accordance with an ear impression, which has a recess (12) on the front side facing away from the auditory canal (40) for loss-proof, custom-fit, and engulfed accommodation of a passive sound insulation filter, and with an active hearing-protection component (20), **characterized in that** the recess (12), having a circular cylindrical indention (12A), to which an additional undercut recess (12B) with a circular cross section and which has a reduced diameter connects on the bottom side, is used as an interface for the active hearing-protection component (20), wherein the active hearing-protection component (20) can be inserted into the recess (12) with a housing portion (20*) formed identically with regard to its overall dimension to the passive sound insulation filter insertable into the hearing-protection earmold, wherein the housing portion (20*) is formed complementarily to the earmold body but with a slight excess to the recess (12), so that the active hearing-protection component (20) can be held in the recess (12) form-fittingly and with elastic force fit.

2. Hearing-protection earmold according to claim 1, **characterized in that** the housing of the active hearing-protection component (20) is essentially cylindrical, wherein the diameter (D20) stays behind the front-side dimensions of the earmold body (10).

3. Hearing-protection earmold according to claim 2, **characterized in that** the axial length (L20) of the housing of the active hearing-protection component (20) corresponds to approximately two times the depth (T12) of the recess (12) in the earmold body (10).

4. Hearing-protection earmold according to one of the claims 1 to 3, **characterized in that** the earmold body (10) surrounds the circular cylindrical indention (12A) with an annular housing portion (14) with preferably constant wall thickness.

5. Hearing-protection earmold according to one of the claims 1 to 4, **characterized in that** the earmold body (10) is made of an elastic silicone rubber material.

6. Hearing-protection earmold according to one of the claims 1 to 5, **characterized in that** in the active hearing-protection component (20), the following components are accommodated and wired together: a volume control (16), a battery (18), a microphone (22), an electronic chip (24) and a miniature sound transducer (26).

7. Hearing-protection earmold according to claim 6, **characterized in that** the miniature sound transducer (26) lies radially within an undercut, conical pin (28) of the active hearing-protection component (20).

8. Hearing-protection earmold according to claim 6 or 7, **characterized in that** a battery compartment cover (30) and a part of the volume control (16) protrude on the front side from the housing of the active hearing-protection component (20).

9. Hearing-protection earmold according to one of the claims 1 to 8, **characterized in that** the active hearing-protection component (20) has an axial length of approximately 10 mm and a diameter of maximal 11 mm.

10. Hearing-protection earmold according to one of the claims 1 to 9, **characterized in that** the passive sound insulation filter can be flushly engulfed in the recess (12).

## Revendications

1. Prothèse otoplastique de protection auditive pour la protection auditive active, avec un corps de prothèse otoplastique (10) travaillé après un moulage de l'oreille, lequel présente, sur son côté frontal opposé au conduit auditif (40), un évidement (12) servant à recevoir de manière imperdable, de manière ajustée avec précision et de manière enfoncée un filtre d'insonorisation passif, et avec une composante de protection auditive active (20), **caractérisée en ce que** l'évidement (12), qui comporte un renfoncement (12A) cylindrique circulaire, auquel se raccorde côté fond un autre évidement (12B) à diamètre réduit, contre-dépouillé, avec une section transversale circulaire, est utilisé en tant qu'interface pour la composante de protection auditive active (20), dans laquelle la composante de protection auditive active (20) peut être insérée dans l'évidement (12) avec une section de boîtier (20*) réalisée, eu égard à ses dimensions extérieures, de manière identique au filtre d'insonorisation passif pouvant être inséré dans la prothèse otoplastique de protection auditive, laquelle section de boîtier est réalisée de manière complémentaire, du moins avec une légère dimension supérieure, par rapport à l'évidement (12) dans le corps de prothèse otoplastique de sorte que la composante de protection auditive active (20) peut être maintenue par complémentarité de forme et avec une force élastique dans l'évidement (12).

2. Prothèse otoplastique de protection auditive selon la revendication 1, **caractérisée en ce que** le boîtier de la composante de protection auditive active (20) est sensiblement cylindrique, dans laquelle le diamètre (D20) reste en dessous des dimensions côté frontal du corps de prothèse otoplastique (10).

3. Prothèse otoplastique de protection auditive selon la revendication 2, **caractérisée en ce que** la longueur axiale (L20) du boîtier de la composante de protection auditive active (20) correspond environ au double de la profondeur (T12) de l'évidement (12) dans le corps de prothèse otoplastique (10).

4. Prothèse otoplastique de protection auditive selon l'une quelconque des revendications 1 à 3, **caractérisée en ce que** le corps de prothèse otoplastique (10) entoure le renfoncement (12A) cylindrique circulaire avec une section de boîtier (14) de forme annulaire avec une épaisseur de paroi de préférence constante.

5. Prothèse otoplastique de protection auditive selon l'une quelconque des revendications 1 à 4, **caractérisée en ce que** le corps de prothèse otoplastique (10) est constitué d'un matériau en caoutchouc siliconé élastique.

6. Prothèse otoplastique de protection auditive selon l'une quelconque des revendications 1 à 5, **caractérisée en ce que** les éléments qui suivent sont logés et câblés les uns aux autres dans la composante de protection auditive active (20) : une commande de réglage de volume (16), une pile (18), un microphone (22), une puce électronique (24) et un transducteur acoustique miniature (26).

7. Prothèse otoplastique de protection auditive selon la revendication 6, **caractérisée en ce que** le transducteur acoustique miniature (26) se trouve de manière radiale à l'intérieur d'un tourillon conique (28) contre-dépouillé de la composante de protection auditive active (20).

8. Prothèse otoplastique de protection auditive selon la revendication 6 ou 7, **caractérisée en ce qu'**un couvercle de compartiment de piles (30) et une partie de la commande de réglage de volume (16) font saillie côté frontal du boîtier de la composante de protection auditive active (20).

9. Prothèse otoplastique de protection auditive selon l'une quelconque des revendications 1 à 8, **caractérisée en ce que** la composante de protection auditive active (20) comporte une longueur axiale d'environ 10 mm et un diamètre de 11 mm au maximum.

10. Prothèse otoplastique de protection auditive selon l'une quelconque des revendications 1 à 9, **caractérisée en ce que** le filtre d'insonorisation passif peut être logé de manière enfoncée en effleurement dans l'évidement (12).
